# EUROPEAN PATENT APPLICATION

(11) **EP 4 604 129 A1**
(43) Date of publication of application: **20.08.2025**
(21) Application number: 24157761.8
(22) Date of filing: 15.02.2024
(51) Int. Cl.: G16B 50/00, G16H 10/60, H04L 9/32, H04L 9/40

(54) **ACCESS SYSTEM FOR GENOMIC DATA SETS AND METHOD FOR OPERATING AN ACCESS SYSTEM**

(71) Applicant: Siemens Healthcare Diagnostics Products GmbH, 35041 Marburg (DE)
(72) Inventor: Guenter, Mélanie, 50674 Köln (DE); Klatt, Torbjörn, 50374 Erftstadt (DE); Masslow, Kay Michael, 53229 Bonn (DE)
(74) Representative: Siemens Healthineers Patent Attorneys

(57) **Abstract**

The invention relates to an access system (1) for genomic data sets (7), comprising a data storage (4), wherein multiple genomic data sets (7) are stored, each genomic data set (7) having associated access permission information associated with a plurality of tenants, an identity storage (3) having a database (8) for associating user identification information, which describes at least a tenant a user belongs to, to the access permission information of the tenants, and a server device (2) connected to the data storage (4) and the identity storage (3), wherein an access software (10) implementing multitenancy for the genomic data sets (7) is running on the server device (2) and is accessible from a plurality of clients (5) of the tenants, in particular web browsers (9) of the clients (5).

## Description

The invention concerns an access system for genomic data sets, comprising a data storage, wherein multiple genomic data sets are stored, each genomic data set having associated access permission information associated with a plurality of tenants. The invention further concerns a computer-implemented method for operating such an access system, a computer program and an electronically readable storage medium.

The analysis and evaluation of genomic and transcriptomic data has become an often-used tool in medical research and clinical diagnosis. Here, in the following, genomic and transcriptomic data will both be addressed by "genomic data". Examples for such genomic data comprise raw sequencing data, for example in the FASTQ format, base sequences mapped to a reference, for example in the BAM or CRAM format, or the sequences of a reference itself, for example in the NIB format. Such genomic data describing a genome sequence is usually stored in source files, which may also be called genomic files.

A major challenge regarding bioinformatic problem-solving in handling genomic data describing genome sequences is the amount of data for each case, that is, each single sample. For example, data regarding a whole genome sequence may have a size of one TB, whole exome data a size of 400 to 600 GB and even targeted panels may still result in file sizes of up to 80 GB. These genomic data are usually read from long-time data storages, for example cloud storages and/or data lakes, via a communication link into a local random access memory of a client, for example for viewing and analysis purposes. Storing the genomic data on a workstation is usually not possible, due to their size.

It is known to store genomic data sets of multiple tenants in the same data storage, for example a cloud storage. Here, a tenant is a group of users sharing common access privileges regarding at least one genomic data set. Sometimes, a tenant may comprise a group of only a single user. To allow a tenant to access genomic data sets registered to him from a client (client computer), different architectures have been proposed in the art. These solutions have to respect both the requirement of mutual exclusion of access regarding the tenants and corporate data governance rules and guidelines, in particular regarding confidentiality. In particular, the genomic data sets of the multiple tenants stored centrally in the data storage have to be logically separated and protected against unauthorized access.

In known architectures, users may access genomic data via genome viewers running on the client. For example, genome viewers which may be run in web pages have been described by Finney, R. and Meerzaman, D. in "Chromatic: WebAssembly-Based Cancer Genome Viewer", Cancer Inform 17 (2018), 1176935118771972, and also by Robinson, J. T. et al. in "igv.js: an embeddable JavaScript implementation of the Integrative Genomics Viewer (IGV)", Bioinformatics 39 (2023), 1. IGV is generally described in an article by Thorvaldsdottir, H. et al, "Integrative Genomics Viewer (IGV): high-performance genomics data visualization and exploration", Brief Bioinform 14 (2013), 178-192. For such local installations, mutual exclusion of access for the multiple tenants is modeled in the operating system by access permissions for file systems. However, using locally installed browsers leads to a high maintenance effort and complexity of the resulting network interactions, in particular regarding authentication and rights management.

It is an object of the current invention to provide remote access to a data storage with genomic data sets of multiple tenants with reduced effort while still respecting access permissions and strict separation of tenants.

This object is achieved by providing an access system, a computer-implemented method for operating an access system, a computer program and an electronically readable storage medium according to the independent claims. Advantageous embodiments are described in the dependent claims.

An access system for genomic data sets according to the invention comprises:
- a data storage, wherein multiple genomic data sets are stored, each genomic data set having associated access permission information associated with a plurality of tenants,
- an identity storage having a database for associating user identification information, which describes at least a tenant a user belongs to, to the access permission information of the tenants,
- a server device connected to the data storage and the identity storage, wherein an access software implementing multitenancy for the genomic data sets is running on the server device and is accessible from a plurality of clients of the tenants, in particular web browsers of the clients. The access software comprises
- an access management software means generating an access token for a genome viewer using authentication information from a client, and
- the genome viewer for outputting genomic data sets to a user based on the access token and a request information describing a request from the client.

The access software implements multitenancy, meaning that for different users of different tenants seeking access from different clients, the access software provides access using the database of the identity storage, wherein, in particular, user identification information for a current user is determined from the authentication information by the access software. This allows determination of the access permission information. Hence, before a genomic data set can be visualized and/or downloaded in the access system, it has to be registered in the database and a user identification information is associated with the permission, that is, access permission information, of the genomic data set. In embodiments, the genomic data set may be associated with a general permission attribute as access permission information, which, in turn, is unambiguously mapped to the user and/or its role, that is, the tenant described by the user identification information.

This architecture eliminates the requirement for a file access permission management modelled by the operating system to separate genomic data sets of different tenants, which would lead to individual, local installation of viewing applications. Hence, central data management and storage is facilitated while still respecting permissions and other security aspects. By using a server-based genome viewer, maintenance effort as well as other associated efforts and costs are reduced.

In particular, genomic data set access permissions can be seamlessly integrated into existing authentication solutions used by the access management software means, since permissions/access rights can easily be derived from authentication mechanism results, in particular comprising user identification information, of the access management software means. Here, generally known concepts and access management software components may be used, which determine group associations and roles from authentication information provided by a client to provide, for example, different services to a user. For example, the access software may comprise further software means accessible through the access management software means, forming a general platform providing different services comprising the genomic data set access.

In this context, preferably, the access management software means is configured to evaluate the authentication information as a login, wherein the authentication information identifies and authenticates the client's user and its tenant. Hence, if a user wants to use the genome viewer, they log in using the authentication information from the client. In particular, no additional logins are required for requests to the genome viewer during a session. Advantageously, known login and authentication processes may also be employed here. For example, authentication information may be provided as explicit login information, for example a user name and a password, but also as OAuth tokens issued by a third party based on a respective login procedure.

Preferably, the data storage is a cloud storage. A cloud storage may provide large storage space, which is particularly suitable to store a large number of genomic data sets.

In particular, the access systems and methods described here may provide a combination of software as a service and cloud storage by including the genome viewer, which allows authentication and permission-based access to centrally stored genomic data sets. The technical solution provides access permission and authentication management, separating centrally stored genomic data associated with different tenants stored centrally and accessed from clients over network connections and the server device.

Generally, remote access by the different clients may be provided via network connections, in particular via the internet. In an especially preferred solution, the server device, in particular the access software, is configured to be accessed via a web browser running on the client. In particular, the server device, in particular the access management software means, is configured to send the output of the genome viewer, in particular comprising genomic data to be output and/or a user interface, to a requesting client for embedding into the output of a web browser. In the web browser, only a light-weight, short script or other web-based client application component, which may be provided with the web site, is required to generate a local viewing application instance, which embeds the genome viewer, in particular its output and interaction therewith, into the web browser, for example a corresponding web page. In preferred embodiments, not only the requested genomic data is output (if the user is permitted to access them), but also the user interface of the genome viewer is shown, allowing the user to interact with the genome viewer as it is running on the client. In this manner, comfortable operation is provided without the requirement of a locally running instance on the server.

Client application components running in the web browser or elsewhere on the clients can generally be understood as part of a distributed access application. The client application components may be understood as part of the access management software means.

The genome viewer may be embedded into the access management software means. This allows less complex interactions between the genome viewer and the access management software means, hence lower computing load on the server device, and a more compact implementation in code as well as in size. However, in other embodiments, it is also conceivable to provide the genome viewer and the access management software means as separate programs communicating with each other.

The access token provided to the genome viewer may be a general "permission to use". The access token, as in principle known, may comprise a session ID and/or an expiry information. The genome viewer checks the validity of the access token and answers requests only of the access token is valid.

Preferably, the access token is a JSON Web Token (JWT). Since JWTs are designed to be compact, access and viewing permissions can be modeled flexibly and fast. Furthermore, using JWT seamlessly integrates itself into existing authentication solutions. For example, both the authentication between client and server device, in particular the login procedure, and the provision of access tokens to the genome viewer may both be based on JWT, in particular even use the same JWT. The access management software means may, for example, use the authentication information for issuing a JWT back to the client, indicating that they are logged in as a certain user of a tenant for an access session. This authentication information may be also used on the server device to issue the access token, in particular also as JWT. However, if a client application component as part of the access management software means directly communicates with the genome viewer, the JWT sent back to the client may be used as the access token.

Preferably, as further discussed below, the payload of the JWT may comprise the user identification information or the user identification information may be derived from the payload.

In embodiments, the access management software means is configured to create an access token for each request to the genome viewer, wherein the access token signs the request as valid. Hence, preferably, each request described by request information from the client may trigger the generation of its own access token, providing a maximum security even during sessions with only one login, but multiple requests. In this manner, communication from the access management software means to the genome viewer is always "signed", i.e. secured. Requests without or with invalid access tokens are denied. In other embodiments, the access token may be valid for a whole access session initiated by a login. In particular, a JWT issued by the access software on the server device may be used to authenticate all requests of the access session. Preferably, however, the JWT created during the login process is valid for the whole access session, however, a new JWT token is determined, in particular for each request, by the access management software means as access token to be communicated to the genome viewer.

In embodiments, the access management software means, in particular on the server device, may be configured to evaluate an incoming request information to the genome viewer coming from a client regarding the permission to access a genomic data set, which is subject to the request, according to the user identification information derived from the authentication information of the client, and to, according to the evaluation result, deny the request or perform one action from an action group, wherein the action group comprises the actions of forwarding the request to the genome viewer and controlling the genome viewer according to the request. The access management software means uses the derived user identification information and the database of the identity storage to check whether the user sending the request from the client is permitted to access the requested genomic data set, in particular belongs to the tenant associated with the genomic data set. If the user is not permitted to access the genomic data set, the request is denied, else, the request, in particular the request information, is either forwarded to the genome viewer or the genome viewer is controlled to fulfil the request, for example by determining control information from the request information. In both cases, the data sent to the genome viewer may be accompanied by the access token, as already discussed above.

Additionally or alternatively, the genome viewer may be configured to evaluate the incoming request information addressed to the genome viewer coming from the client, the request information relating to access to a genomic data set, wherein evaluation comprises checking if a tenant, which is described by user identification information provided as part of or derived from the incoming request and/or the access token and determined using the authentication information, is permitted to access the genomic data set specified by request information according to the database. If the result of the check is that the user is not permitted to access this particular genomic data set, the request is denied. Preferably, the user identification information (or information from which it can be derived) is passed to the genome viewer via the access token, in particular as payload of a JWT.

In some embodiments, at least for requests directed to specific genomic data sets, both the access management software means on the server device and the genome viewer may be configured to check the permissions to access certain genomic data sets using the database. In this manner, separation and hence data security can be improved. It is noted that a request may also comprise retrieving a list of (not yet specific) accessible genomic data sets, where, preferably, the list for a certain user may be provided by the genome viewer, checking permissions in the database. However, also in such a case, the list may be checked for plausibility by the access management software means, also using the database.

It is generally noted that the server device and each of the clients may comprise at least one processor and at least one storage means. In embodiments, the identity storage and/or the data storage may be comprised by the storage means of the server device, such that connections are internal to the server device, which may be a cloud server and/or comprise a web server. However, the identity storage and/or the data storage may also be external to the server device and connected using, preferably, physical and/or wireless connections.

In a computer-implemented method for operating an access system for genomic data sets, according to the invention, the access system comprises
- a data storage, wherein multiple genomic data sets are stored, each genomic data set having associated access permission information associated with a plurality of tenants,
- an identity storage having a database for associating user identification information, which describes at least a tenant a user belongs to, to the access permission information of the tenants,
- a server device connected to the data storage and the identity storage, wherein an access software implementing multitenancy for the genomic data sets is executed on the server device and is accessible from a plurality of clients of the tenants, in particular web browsers of the clients. The access software comprises
- an access management software means, which, at least once for each access session from a client, generates an access token for a genome viewer using authentication information from the client, and
- the genome viewer, which outputs genomic data sets to a user based on the access token and a request information describing a request from the client.

Here, the access software evaluates the authentication information to determine a user identification information, which, in turn, is used to check access permissions in the database to establish multitenancy. The access management software means and/or the genome viewer may perform the check, as discussed above. All features and remarks regarding the access system can also be applied to the method and vice versa.

In particular, for each access session,
- the access management software means performs a login process based on the authorization information to start the access session,
- for each access session or for each request, described by incoming request information, during the access session, the access management software means generates the access token for the genome viewer, in particular a JSON web token,
- for each request, described by incoming request information, relating to a genomic data set, the genome viewer, after at least checking the validity of the access token, retrieves the genomic data set from the data storage, wherein the output of the genome viewer is sent to the client of the access session, in particular for output in a web browser of the client.

In an embodiment,
- in the login process, a JSON web token (JWT) is generated on the server device, the JWT comprising a payload which comprises or allows deriving of the user identification information determined from the authentication information, and
- the generated JWT is used as the access token for each request of the access session.

This allows optimal integration into the authentication procedure.

Preferably, however, the JWT generated in the login process is used by the client to authenticate to the access management software means on the server device during the access session, while the access management software means generates a new JWT, in particular based on the login process JWT and/or for each request, as the access token for the genome viewer. The access token may comprise a payload which comprises or allows deriving of the user identification information determined from the authentication information. The access token is then communicated to the genome viewer with or immediately before forwarding the request information. In particular, the access management software means and the genome viewer may use different authentication directories, wherein the database of the identity storage may also provide the authentication directory for the genome viewer. Security may be improved by using different tokens, in particular when also using different authentication sources.

A computer program according to the invention comprises program means such that, when the computer program is executed on a server device of an access system, the server device is caused to perform a method according to the invention. The computer program may be or comprise the access software. The computer program may be stored on an electronically readable storage medium according to the invention, which hence comprises control information stored thereon, the control information comprising a computer program according to the invention and being configured such that, when the electronically readable storage medium is used in a server device of an access system, the server device is configured to perform a method according to the invention.

Other objects and features of the present invention will become apparent from the following detailed description considered in conjunction with the accompanying drawings. The drawings, however, are only principle sketches designed solely for the purpose of illustration and do not limit the invention. The drawings show:
- Fig. 1: an embodiment of an access system according to the invention, and
- Fig. 2: a flowchart of an embodiment of a method according to the invention.

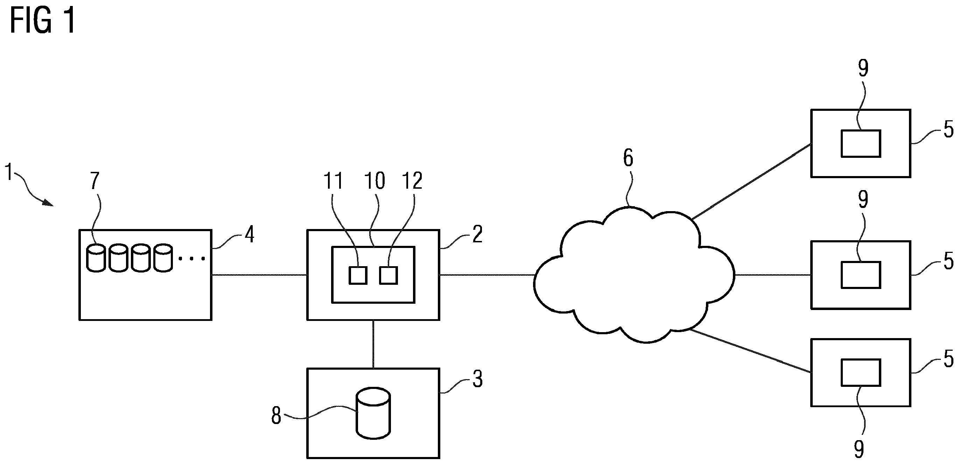

Fig. 1 is a functional drawing of an access system 1 according to the invention. The access system 1 comprises a server device 2, an identity storage 3 and a data storage 4, which are interconnected. The server device 2 comprises at least one processor and at least one storage means. The storage means may, in embodiments, comprise the identity storage 3 and/or the data storage 4. The server device 2 may be a cloud server and/or the data storage 4 may be a cloud storage. The server device 2 comprises a web server.

Using the web server, multiple clients 5 (client computers) associated with a plurality of tenants may access the server device 2 via network connections in a network 6, which may be or comprise the internet, using web browsers 9.

In the data storage 4, genomic data sets 7 of the different tenants are stored. Upon storing a genomic data set 7 into the data storage 4, a permission to access is associated with it in the form of a permission access information. To register the genomic data set 7, the permission access information is unambiguously linked to a user identification information, which describes the tenant associated with the genomic data set 7, in a database 8 stored in identity storage 3.

If a user of a tenant wants to access a genomic data set 7, he uses the respective web browser 9 to call up a web page from the server device 2 to use an access software 10 running on the server device 2. In this process, a client application component may be downloaded to the client 5 and executed in the web browser 9. The access software 10 comprises an access management software means 11 and a genome viewer 12. The genome viewer 12 may be embedded into the access management software means 11 or a component thereof, and/or the client application component may be a part of the access management software means 11. The user interface and the output of the genome viewer 12 are embedded into the web site shown in the web browser 9 of the client 5. The access software 10 is a multitenancy application allowing access from a plurality of users at clients 5 via a centrally running genome viewer 12 while respecting access permissions and authenticating each user.

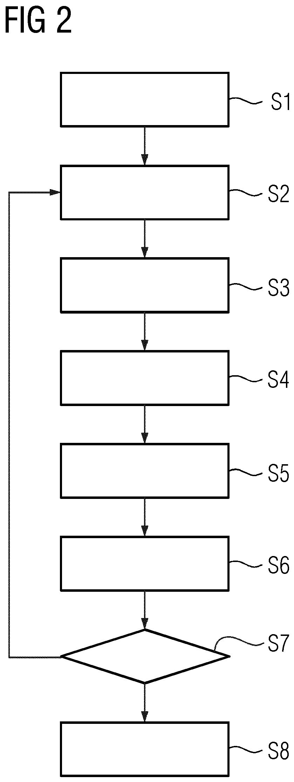

Fig. 2 shows a flowchart of an embodiment of a method implemented in the access system 1, wherein all steps on the server side are performed by the access software 10.

In a step S1, an access session of a user from one of the clients 5 begins after the user has opened the web site for accessing the access system 1 and chosen to log in. To this end, authentication information of the user is received by the access management software means 11 on the server device 2 in login step S1. The authentication information may comprise login credentials like user name and password, but may also certify that the user is already logged in at another server. In the latter case, the authentication information may comprise an OAuth token. The authentication information is checked by the access management software means 11 on the server device 2 and, if the user is successfully authenticated, the access session is started. The successful login is completed by providing a JWT token back to the client 5, in particular the client application component.

The authentication information allows to determine the tenant a user belongs to, meaning that the access software 10 can determine user identification information from the authentication information and check permissions to access genomic data sets 7. It is noted that, while client application components may be a part of the access management software means 11, the authorization information is evaluated and the JWT token of the login process is issued on the server device 2.

For example, a menu may be displayed in web browser 9. The user can now choose that he wants to access genomic data sets 7, such that the user interface of the genome viewer 12 can be displayed in web browser 9. In other words, the output of the genome viewer 12 for this user is output embedded into the web page, such that the user can interact and generate request information describing a request as a result of the interaction. In an access session after the login in step S1, multiple requests can be processed, as further described with respect to steps S2 to S8.

In a step S2, request information regarding a request relating to a genomic data set 7 is received by the access management software means 11. If access to a specific genomic data set 7 is requested, the access management software means 11 may optionally check access permission on the server side using the database 8 (using user identification information determined from the authentication information) and deny the request if access by this user is not permitted.

In any case, in a step S3, the access management software means 11 either, in particular in the case of the client application component, uses the JWT provided as result of the login procedure in step S1 as an access token, or, preferably, a new JWT is generated as an access token using the authentication information and optionally the request information on the server device 2. The access token has a validity interval. In this embodiment, the payload of the access token comprises the user identification information or at least data, from which the user identification information of the current user can be derived. In other words, the JWT used as access token allows determining the permission access information for the tenant of the current user. The access token and the request information are sent to the genome viewer 12.

In a step S4, the genome viewer 12, after receiving the JWT used as access token and checking its validity, extracts the identity of the user, in particular their affiliation to a tenant. In other words, the user identification information of the respective user is determined from the access token. If access is not permitted according to the database 8, the request is denied. Else, in a step S5, the request is fulfilled, in particular by loading the requested genomic data from the persistence layer, that is, the data storage 4 and updating the output.

In this embodiment, the genome viewer 12 was upgraded by adding additional authentication and permission checking functionalities, allowing secure and authorized, remote access to multiple storage accounts in the data storage 4, in particular cloud storage.

In a step S6, the output of the genome viewer is sent to the respective client 5 for outputting in the web browser 9. In a step S7, it is checked if further requests are expected or if the access session has expired (user-initiated or time out). If further requests are to be handled, it is returned to step S2, else the access session ends in step S8.

Of course, multiple users can use the access system 1 simultaneously.

Although the present invention has been described in detail with reference to the preferred embodiment, the present invention is not limited by the disclosed examples from which the skilled person is able to derive other variations without departing from the scope of the invention.

Independent of the grammatical term usage, individuals with male, female or other gender identities are included within the term.

### List of reference numbers

- 1: access system
- 2: server device
- 3: identity storage
- 4: data storage
- 5: client
- 6: network
- 7: genomic data set
- 8: database
- 9: web browser
- 10: access software
- 11: access management software means
- 12: genome viewer

- S1-S8: Step

## Claims

1. Access system (1) for genomic data sets (7), comprising
- a data storage (4), wherein multiple genomic data sets (7) are stored, each genomic data set (7) having associated access permission information associated with a plurality of tenants,
- an identity storage (3) having a database (8) for associating user identification information, which describes at least a tenant a user belongs to, to the access permission information of the tenants,
- a server device (2) connected to the data storage (4) and the identity storage (3), wherein an access software (10) implementing multitenancy for the genomic data sets (7) is running on the server device (2) and is accessible from a plurality of clients (5) of the tenants, in particular web browsers (9) of the clients (5),
- wherein the access software (10) comprises
- an access management software means (11) generating an access token for a genome viewer (12) using authentication information from a client (5), and
- the genome viewer (12) for outputting genomic data sets (7) to a user based on the access token and a request information describing a request from the client (5) .

2. Access system according to claim 1, **characterized in that** the access software (10) is configured to determine user identification information from the authentication information.

3. Access system according to claim 1 or 2, **characterized in that** the access token is a JSON Web Token.

4. Access system according to one of the preceding claims, **characterized in that** the access management software means (11) is configured to create an access token for each request to the genome viewer (12), wherein the access token signs the request as valid.

5. Access system according to one of the preceding claims, **characterized in that** the access management software means (11) is configured to evaluate an incoming request information to the genome viewer (12) coming from a client (5) regarding the permission to access a genomic data set (7), which is subject to the request, according to the user identification information derived from the authentication information of the client (5), and to, according to the evaluation result, deny the request or perform one action from an action group, wherein the action group comprises the actions of forwarding the request to the genome viewer (12) and controlling the genome viewer (12) according to the request.

6. Access system according to one of the preceding claims, **characterized in that** the genome viewer (12) is configured to evaluate an or the incoming request information to the genome viewer (12) coming from a or the client (5), the request information relating to access to a genomic data set (7), wherein evaluation comprises checking if a tenant, which is described by user identification information provided as part of or derived from the incoming request and/or the access token and determined using the authentication information, is permitted to access the genomic data set (7) specified by request information according to the database (8).

7. Access system according to one of the preceding claims, **characterized in that** the access management software means (11) is configured to evaluate the authentication information as a login, wherein the authentication information identifies and authenticates the client's user and its tenant.

8. Access system according to one of the preceding claims, **characterized in that** the server device (2), in particular the access management software means (11), is configured to send the output of the genome viewer (12), in particular comprising genomic data to be output and/or a user interface, to a requesting client (5) for embedding into the output of a web browser (9).

9. Access system according to one of the preceding claims, **characterized in that** the data storage (4) is a cloud storage.

10. Method for operating an access system (1) for genomic data sets (7), the access system (1) comprising
- a data storage (4), wherein multiple genomic data sets (7) are stored, each genomic data set (7) having associated access permission information associated with a plurality of tenants,
- an identity storage (3) having a database (8) for associating user identification information, which describes at least a tenant a user belongs to, to the access permission information of the tenants,
- a server device (2) connected to the data storage (4) and the identity storage (3), wherein an access software (10) implementing multitenancy for the genomic data sets (7) is executed on the server device (2) and is accessible from a plurality of clients (5) of the tenants, in particular web browsers (9) of the clients (5),
- wherein the access software (10) comprises
- an access management software means (11), which, at least once for each access session from a client (5), generates an access token for a genome viewer (12) using authentication information from the client (5), and
- the genome viewer (12), which outputs genomic data sets (7) to a user based on the access token and a request information describing a request from the client (5) .

11. Method according to claim 10, **characterized in that**, for each access session,
- the access management software means (11) performs a login process based on the authorization information to start the access session,
- for each access session or for each request, described by incoming request information, during the access session, the access management software means (11) generates the access token for the genome viewer (12), in particular a JSON web token,
- for each request, described by incoming request information, relating to a genomic data set (7), the genome viewer (12), after at least checking the validity of the access token, retrieves the genomic data set (7) from the data storage (4), wherein the output of the genome viewer (12) is sent to the client (5) of the access session, in particular for output in a web browser (9) of the client (5).

12. Computer program, which, when executed on a server device (2) of an access system (1), causes the server device (2) to perform the steps of a method according to claim 10 or 11.

13. Electronically readable storage medium, on which a computer program according to claim 12 is stored.
